# EUROPEAN PATENT SPECIFICATION

(11) **EP 0 671 976 B1**
(45) Date of publication and mention of the grant of the patent: **04.08.1999**
(21) Application number: 94900962.5
(22) Date of filing: 02.12.1993
(51) Int. Cl.: B01J 23/06, B01J 23/80, B01J 32/00, B01J 35/10, C07C 1/04

(54) **Fischer-Tropsch reaction with a catalyst comprising a zinc oxide composition**
Fischer-Tropsch Reaktion unter Verwendung von Katalysatoren, welche eine Zinkoxydzusammensetzung enthalten
Réaction Fischer-Tropsch avec un catalyseur, contenant un composition d'oxyde de zinc

(30) Priority: 04.12.1992 GB 9225372
(43) Date of publication of application: 20.09.1995
(73) Proprietor: BP AMOCO P.L.C., London EC2M 7BA (GB)
(72) Inventor: NAY, Barry, Surrey GU21 3HB (GB)
(74) Representative: Wilson, Michael John
(86) International application number: GB9302479
(87) International publication number: WO9413400

(56) References cited:
- EP-A- 0 089 587
- EP-A- 0 117 496
- EP-A- 0 261 870
- US-A- 4 107 091
- US-A- 4 340 745
- Derwent's abstract No. 90606 D/49, week 8149,ABSTRACT OF SU 810 253, (AS LATV ORGAN SYNTH), 1981-03-07

## Description

This invention concerns Fischer-Tropsch reactions in the presence of catalysts comprising zinc oxide compositions.

Zinc oxide is used in catalysts as a support for catalytically active metals such as cobalt in Fischer-Tropsch catalysts (see e.g. our EP-A-261870). One problem with it is that it suffers shrinkage and sintering on heating to high temperatures, which may be those of the catalyst use or its regeneration. This shrinkage happens progressively with time and results in loss of activity.

We have discovered that the copresence of a trivalent metal of Group IIIB of the Periodic Table of the Elements, especially aluminium in a zinc oxide composition of high surface area can increase the surface area of the zinc oxide and/or reduce the loss of surface area on long term heating. Cobalt-containing catalysts supported on said composition can be of high activity with good retention of that activity in spite of prolonged heating in use and/or regeneration.

The present invention provides a method of performing a Fischer-Tropsch reaction to convert a mixture of carbon monoxide and hydrogen to hydrocarbons which comprises passing, under Fischer-Tropsch conditions, said mixture over a catalyst composition comprising a Group VIII metal supported on a zinc oxide composition, which zinc oxide composition after calcination at 300°C for 5hr has a surface area of at least 30m²/g, said zinc oxide composition consisting essentially of zinc oxide and a trivalent metal of Group IIIB of the Periodic Table, said metal being in atomic percentage to the zinc of 0.4-15%, and in the form of metal and/or metal oxide and/or a compound thermally decomposable thereto and/or a compound, e.g. a spinel, with the zinc oxide.

The zinc oxide may be made by precipitation from an aqueous solution of a soluble zinc salt, such as zinc nitrate or chloride, by mixing with a weak base, such as ammonium hydroxide, carbonate or bicarbonate, or a tetraalkylammonium hydroxide or an organic primary, secondary or tertiary amine, at a temperature of 0°C to the boiling point, usually at less than 50°C such as 0-50°C, and usually at pH5.5-8.5 followed by separation of the zinc hydroxide and/or carbonate precipitate, e.g. by filtration, then washing, e.g. with water, especially deionised water, to remove extraneous soluble ions, drying, e.g. at 100-200°C and then usually heating (calcination), e.g. in an inert atmosphere or an atmosphere comprising molecular oxygen, such as nitrogen or air, at 200-600°C for 6-1 hours such as 3hr at 300°C to produce the zinc oxide. This zinc oxide may itself have a surface area, determined by BET measurements with nitrogen (as are all surface areas herein), of at least 20 m²/g, such as 40-80m²/g.

The trivalent metal is a metal of Group IIIB of the Periodic Table; as used in this specification the Periodic Table is that given in "Advanced Inorganic Chemistry" by F.A.Cotton and G.Wilkinson, J.Wiley, New York, 4th Ed. 1980. The trivalent metal of Group IIIB of the Periodic Table is, especially gallium or indium but most particularly aluminium. The invention will be particularly described with reference to aluminium but corresponding processes adapted as necessary may be used for the other trivalent metals.

The aluminium may be present in the zinc oxide composition, following impregnation with aluminium of the above zinc oxide, whether in the form before or after calcination, or coprecipitation of aluminium with the zinc oxide. In the impregnation route, the above zinc oxide may be treated with an aqueous or organic solution of an aluminium compound, especially a thermally decomposable one, such as aluminium nitrate or a carboxylate such as an alkanoate, such as acetate or naphthenate or a dicarbonyl complex such as acetyl acetonate; preferably there is used an anhydrous solution in an organic solvent, such as an alkanol, e.g. of 1-6 carbons, such as methanol or ethanol or a ketone, such as a dialkylketone, e.g. of 3-6 carbons such as acetone or an ether such as a dialkylether, e.g. of 4-8 carbons, such as diethyl ether or a cyclic ether, e.g. of 4-8 carbons such as tetrahydrofuran, or a liquid paraffin hydrocarbon. The impregnation may be by the incipient wetness technique. After impregnation, the residual water or solvent is evaporated to leave an impregnated solid which is an example of the above specified precursor, and which is usually calcined, e.g. as described above. Preferably however the aluminium is incorporated by coprecipitation. The precipitation technique described above for production of zinc oxide may be modified by the presence of a water soluble aluminium salt, such as the nitrate, chloride or another as described above, in the aqueous solution of zinc salt before mixing with the weak base, e.g. to pH 5.5-10, followed by precipitation, washing, drying and calcining as before. The precipitation or impregnation of the zinc oxide and the zinc oxide composition may be performed in a batch or continuous manner and preferably with agitation, e.g. stirring and especially with maintenance of the pH at 5.5-10 such as 5.5-7.5. Preferably an aqueous solution of the zinc salt and an aqueous solution of the water soluble aluminium salt (or other one of said metal salts), are added continuously to an agitated aqueous solution of said weak base while keeping the pH at 5.5-10 especially 5.5-8.5 to produce a coprecipitate which is separated. Preferably the zinc salt and aluminium (or salt of said other metal) are premixed so the aqueous solution added to the base contains both salts.

The zinc oxide composition of the invention, alter calcination e.g. in air at 300°C for 5hrs, has a surface area of at least 30m²/g, preferably at least 80m²/g, e.g. 30-200 or 80-150m²/g. At least some (and preferably substantially all) the trivalent metal, especially aluminium, is usually present in the zinc oxide composition in the form of a spinel, whose presence can be determined by X-ray crystallography, so preferably the composition comprises a mixture of zinc oxide and said spinel.

The atomic amount of the trivalent metal is 0.4-15% (relative to the zinc in the zinc oxide composition), preferably 0.5-10% such as 0.9-6% especially 1.4-5%. The weight amount of trivalent metal especially aluminium or gallium in the zinc oxide composition is 0.5-7.5% by weight (as metal expressed by weight of zinc oxide), particularly 0.7-5% and is especially 0.8-2%. Amounts of the metal especially expressed as aluminium oxide, may be 1.0-15% e.g. 1.5-10% and especially 1.6-4% by weight.

The zinc oxide composition may be made as above in the form of a powder, e.g. of 1-100 microns average diameter, but preferably for use as a catalyst support it is in the form of granules, e.g. of 100-1000 microns average diameter. The granules may be made, optionally after wetting of powder with water or solvent, by compression and granulation and sieving; the powder may be the calcined product made as above, but preferably the powder is the dried product before calcination, so that the calcination is performed on the granulated product. The zinc oxide compositions of the invention are usually of higher compression strength than those without the extra trivalent metal.

The zinc oxide composition, especially after calcination under conditions as described before, are used as catalyst supports, for one or more Group VIII metals, e.g. nickel or preferably iron, but especially cobalt. The Group VIII metal may be present as an oxide and/or a compound thermally decomposable thereto. The use of cobalt will be described further, but the other Group VIII metals may be used instead. The cobalt is dispersed in the zinc oxide compositions which may be in the form of the powder, but preferably as granules. The dispersion may be substantially throughout the zinc oxide composition solid or substantially only at its surface. The dispersion may be achieved in a manner known per se for dispersion of cobalt onto catalyst supports, especially zinc oxide catalyst supports, e.g. as described in EP-A-261870, usually but not by coprecipitation of cobalt with the zinc and trivalent metal e.g. aluminium. The former dispersion may be achieved by immersion of the zinc oxide composition solid in a solution of the cobalt compound; the solution is preferably in an organic solvent, e.g. as described above. The cobalt compound, when supported on zinc oxide, is thermally decomposable to a cobalt oxide. It may be an inorganic salt, e.g. cobalt nitrate or a carboxylate, preferably an alkanoate, e.g. with 1-18 carbons in the alkanoate group such as acetate or octanoate or naphthenate, or a co-ordination complex such as a carbonyl or an organic di-carbonyl complex such as with acetylacetonate. The surface dispersion of cobalt on the zinc oxide composition may be achieved by spray coating the composition with a solution of the cobalt compound with evaporation of the solvent, e.g. by spraying the solution onto the solid composition at a temperature of at least 100°C. The coating may be performed a sufficient number of times to build up the desired cobalt level and to the desired depth of impregnation. After the impregnation, the impregnated zinc oxide composition is usually dried, e.g. at 100-250°C, followed by calcination, e.g. by heating at 250-600°C for 1-8 hrs e.g. at 400°C for 2-4hrs.

The cobalt-containing zinc oxide composition can have 1-300% Group VIII metal (expressed in g. atoms of metal on the basis of the moles of zinc oxide in the composition), in particular 2-200% and especially 5-70% such as 10-30%. The composition may contain 3-40%, e.g. 3-25% of the Group VIII metal (as metal on the basis of the total weight of the composition). If desired, a promoter or promoters may also be present, e.g. Zr, Ti, Ru and/or Cr and these are preferably impregnated in the zinc oxide composition, especially coimpregnated with the cobalt or other Group VIII metal. The catalyst compositions containing at least one Group VIII metal are preferably substantially free from alkali metal and alkaline earth metal, such as calcium, and are thus usually substantially free of calcium cements, e.g. calcium aluminates. Preferably however the catalyst compositions contain the Group VIII metal, especially cobalt as substantially the only transition metal, and especially consist substantially essentially of Zn and O atoms and of atoms of the trivalent metal, and atoms of the Group VIII metal especially cobalt.

The catalyst compositions of the invention containing the Group VIII metal or metals are used in a Fischer-Tropsch reaction in a manner known per se for Group VIII catalysts in said reaction. Generally, the catalyst, after granulation, compression or pelletization and crushing, is treated by passing over it a mixture of hydrogen and carbon monoxide, especially in a molar ratio of 0.5-6:1, e.g. 1.5-2.5:1 or 1.7-2.5:1, such as synthesis gas. The reaction may be performed in a fixed bed, fluidised bed or slurry phase reactor or a two or three phase fluidised bed reactor, and may be a batch semi-continuous or continuous operation. It may be performed under an elevated pressure, e.g. of 0.1-10MPa, such as 1-5MPa and at an elevated temperature, e.g. of 160-350°C, preferably 180-250°C especially 185-210°C. The gas hourly space velocity GHSV for continuous operation is usually at least 100, e.g. 100-25000hr⁻¹ especially 500-10,000hr⁻¹. Before use in the process, the catalyst is usually activated to form at least some Group VIII metal (or lower valency metal oxide) by reduction with hydrogen or a hydrogen-rich mixture with carbon monoxide (all optionally diluted with nitrogen) for example at a temperature of 150-500°C, e.g. 200-300°C for a period of at least 5hrs such as 5-24hrs. The activation may involve heating to the above temperatures first in an inert atmosphere, e.g. of nitrogen and then, preferably alter cooling, heating in the reducing atmosphere. The Fischer-Tropsch process is particularly selective to produce hydrocarbons, especially with at least 5 carbons such as 5-60 carbons and in particular waxes. The process can have conversions of carbon monoxide and of hydrogen each of at least 50% especially at least 60% and selectivities to hydrocarbons of at least 3 carbons of at least 70% especially at least 80%, and to hydrocarbons of at least 5 carbons of at least 60% e.g. at least 70%. The selectivities to carbon dioxide and C₂-hydrocarbons are each preferably below 1.5%, while the selectivity to methane is usually less than 10% such as 5-10%. After use, the catalyst can be regenerated, e.g. by heating at 300-600°C, e.g. 400-600°C in the presence of a gas comprising molecular oxygen, and may then be reductively activated as described above.

The catalyst compositions have high initial surface area, usually substantially the same as for the zinc oxide compositions and have, after heating for 10 hours, such as 10-60 hours, at 500°C, e.g. during regeneration or activation, a surface area of at least 30m²/g such as 30-60, or especially 40-60m²/g.

The invention is illustrated in the following Examples.

### SUPPORT PREPARATION

### Example (a)

Zinc nitrate hexahydrate (182.8g) and aluminium nitrate nonahydrate (3.49g) were dissolved in deionised water (0.46dm³) and pumped at 50cm³ min⁻¹ into a rapidly stirred solution of ammonium bicarbonate (291.5g) dissolved in deionised water (2.31dm³) to give a suspension at pH 7-8. The white co-precipitate was filtered and washed once by slurrying with deionised water (3.0dm³) for 30 minutes. The slurry was refiltered, and the white filter cake dried overnight (16h) at 120°C in air to leave a zinc oxide composition containing 0.5% Al (expressed as Al by weight of zinc oxide). X-ray diffraction on the composition showed the presence of zinc oxide and ZnAl₂O₄ (gahnite, a spinel in significant amounts).

### Example (b)

The process of Example (a) was repeated but with about double the amount of aluminium nitrate nonahydrate (namely 7.02g). The product was a zinc oxide composition containing 1.0% aluminium (expressed as Al by weight of zinc oxide).

### Example (c)

The process of Example (b) was repeated but with the aluminium nitrate replaced by gallium nitrate hexahydrate (1.835g) to give a zinc oxide composition containing 0.82mol% gallium (based on moles of ZnO).

### Example (d)

The effect of heat on the surface areas of the compositions of Examples (a)-(c) were tested initially (after heating in air for 3hrs at 300°C) and then after subsequently heating at 500°C for 5, 24 and 60 hrs. The surface area was determined by BET measurements with nitrogen. The results were compared with those on a zinc oxide Composition (A) made as in Example (a) but without the aluminium.

The results were as follows:-

| **Composition** | **Surface Area (m**^{**2**}**/g)** | | | |
|---|---|---|---|---|
| | **Initially** | **After 5hrs** | **After 24 hrs** | **After 60 hrs** |
| A | 55 | 14 | | <5 |
| Example (a) | 84 | 52 | 37 | 34 |
| Example (b) | 104 | 62 | 52 | 47 |
| Example (c) | 58 | 22 | 21 | 19 |

The products of Ex (a)-(c) had a higher compression strength than that of Composition A.

### PREPARATION OF CATALYST

### Example (e)

Cobalt nitrate nonahydrate (54.9g) dissolved in deionised water (200cm³) was slowly added to the powder of the composition of Example (a) (100g) which had previously been calcined in air at 300°C for 3hrs. The slurry/powder obtained was throughly mixed, and then dried overnight at 90°C in air. The dry powder was ground to <500 microns, and calcined in air by increasing the temperature at a rate of 100°C per hour to 400°C, at which it was kept for 3 hrs. The resulting powder was pressed to form tablets under a pressure of 9 tonnes which were crushed and then the crushed granules sieved to 250-500 microns. The product was a catalyst composition B containing 10% cobalt (as Co, based on the weight of the composition of Example (a)).

### USE OF THE CATALYST IN THE FISCHER-TROPSCH PROCESS

### Example 1

The catalyst composition B (10cm³:13.58g) was loaded into a microreactor and reduced in hydrogen at atmospheric pressure with the temperature increased at a rate of 2°C min⁻¹ to 300°C, which temperature was then held for 10hrs. The reactor was then cooled to less than 60°C and syngas (CO:H₂ in a molar ratio of 2.07:1, plus 20% nitrogen) was introduced to the reactor. The pressure in the reactor was increased to 30 bar, and the applied temperature increased slowly to 180°C. The temperature was then increased in small increments until >70% CO conversion was attained and periodic mass balances were carried out to check the conversion and productivity. The average applied and bed temperatures were 197-8°C at least after 73hrs when the conversion was 73.08% and productivity to C₅-hydrocarbons was 117.49 g/lbed/hr, and after 140.1 hrs when the conversion was 67.48% and the productivity to C₅₊ -hydrocarbons was 111 g/lbed/hr.

### Examples 2 and 3

The process of Example 1 was repeated with two levels of gallium nitrate addition, namely 4.62g and 2.32g (for Example 2 and 3 respectively) to give zinc oxide compositions containing 2.06 mol% and 1.0 mol% Ga (based on mols of ZnO). The surface area of the compositions and the effect of heat thereon was determined as in Example (d).

The results are as follows:

| | | **Surface Area m**^{**2**}**/g** | |
|---|---|---|---|
| **Composition** | **Initially** | **After 24hrs** | **After 60 hrs** |
| Example 2 | 94 | 49 | 47 |
| Example 3 | 88 | 36 | 31 |

### Comparative Examples

(i) The cobalt impregnation process of Example (e) was repeated using the composition A (zinc oxide without added aluminium) with a different amount of cobalt to give catalyst composition C containing 16% cobalt (as Co, based on the weight of the zinc oxide). The surface area of the cobalt impregnated product was determined as described in Ex. (d) before and after heating. The conditions of heating and results were as follows.

| **Surface Area** | **Composition C** |
|---|---|
| Surface Area m²/g | |
| Product | - |
| after 6 hr calcination at 300°C | 136 |
| after 60 hr calcination at 500°C | 13 |
| Particle size (micron) of product as made | 250-500 |

(ii) Composition C was tested in the Fischer-Tropsch process under conditions similar to those described in Ex 1 and the productivity of C₅₊ -hydrocarbons was determined.

The results were as follows, compared to those in Ex 1: the relative productivity means the percentage of the productivity after the longer hours on stream to the productivity after the shorter hours.

| | **Composition C** | | **Example 1** | |
|---|---|---|---|---|
| Hours in stream | 160 | 310 | 73 | 140 |
| Relative Productivity | 100 | 77 | 100 | 95 |

The Composition of Ex 1 also had a higher compression strength than that of Composition C.

## Claims

1. A method of performing a Fischer Tropsch reaction to convert a mixture of carbon monoxide and hydrogen to hydrocarbons which comprises passing, under Fischer Tropsch conditions, said mixture over a catalyst composition comprising a Group VIII metal supported on a zinc oxide composition, which zinc oxide composition after calcination at 300°C for 5 hours has a surface area of at least 30 m²/g, said zinc oxide composition consisting essentially of zinc oxide and a trivalent metal of Group IIIB of the Periodic Table, said metal being in atomic percentage to the zinc of 0.4-15%, and in the form of metal and/or metal oxide and/or a compound thermally decomposable thereto, and/or a compound with said zinc oxide.

2. A method according to claim 1 wherein the trivalent metal is gallium or aluminium.

3. A method according to claim 2 wherein the metal is aluminium.

4. A method according to claim 2 wherein the aluminium or gallium is present at least partly in the form of a spinel with said zinc oxide.

5. A method according to any one of the preceding claims wherein the zinc oxide composition has a surface area of 80-150 m²/g after calcination at 300°C for 5 hours.

6. A method according to any one of the preceding claims wherein the atomic proportion of the metal of Group IIIB of the Periodic Table to zinc in the zinc oxide composition is 1.4 to 5%.

7. A method according to any one of the preceding claims wherein the catalyst composition is obtained by the steps comprising in turn:-
(i) forming a zinc oxide composition, which after calcination at 300°C for 5 hours has a surface area of at least 30 m²/g, said zinc oxide composition consisting essentially of zinc oxide and a trivalent metal of Groups IIIB of the Periodic Table, said metal being in atomic percentage to the zinc of 0.4-15%, and in the form of metal and/or metal oxide and/or a compound thermally decomposable thereto, and/or a compound with said zinc oxide, and thereafter
(ii) dispersing the Group VIII metal in the zinc oxide composition.

8. A method according to claim 7 wherein the zinc oxide is made by precipitation from an aqueous solution of a soluble zinc salt.

9. A method according to claim 8 wherein the zinc oxide is thereafter calcined.

10. A method according to either claim 8 or claim 9 wherein the zinc oxide is impregnated with the Group IIIB metal.

11. A method according to claim 8 wherein the zinc oxide and the Group IIIB metal are coprecipitated.

12. A method according to any one of claims 7 to 11 wherein the Group VIII metal is dispersed on the zinc oxide composition by impregnation.

13. A method according to any one of claims 7 to 12 wherein the catalyst before use in the Fischer Tropsch reaction is activated to form at least some Group VIII metal (or lower valency metal oxide) by reduction with hydrogen or a hydrogen rich mixture with carbon monoxide.

14. A method according to any one of claims 7-13 wherein the Group VIII metal is cobalt.

15. A method according to any one of the preceding claims wherein Fischer Tropsch conditions are an elevated pressure in the range 0.1 to 10 MPa and an elevated temperature in the range 180 to 250°C.

## Patentansprüche

1. Verfahren zur Ausführung einer Fischer-Tropsch-Reaktion, um ein Gemisch von Kohlenmonoxid und Wasserstoff zu Kohlenwasserstoffen umzuwandeln, das Leiten des Gemisches unter Fischer-Tropsch-Bedingungen über eine Katalysatorzusammensetzung, umfassend ein Metall der Gruppe VIII, getragen auf einer Zinkoxidzusammensetzung, umfaßt, wobei die Zinkoxidzusammensetzung nach Calcinierung bei 300°C für 5 Stunden eine Oberfläche von mindestens 30 m²/g aufweist, die Zinkoxidzusammensetzung im wesentlichen aus Zinkoxid und einem dreiwertigen Metall der Gruppe IIIB des Periodensystems besteht, wobei das Metall in Atomprozent zu dem Zink mit 0,4-15% und in Form von Metall und/oder Metalloxid und/oder einer Verbindung, die thermisch dazu zersetzbar ist und/oder einer Verbindung mit dem Zinkoxid vorliegt.

2. Verfahren nach Anspruch 1, wobei das dreiwertige Metall Gallium oder Aluminium ist.

3. Verfahren nach Anspruch 2, wobei das Metall Aluminium ist.

4. Verfahren nach Anspruch 2, wobei das Aluminium oder Gallium mindestens teilweise in Form eines Spinells mit dem Zinkoxid vorliegt.

5. Verfahren nach einem der vorangehenden Ansprüche, wobei die Zinkoxidzusammensetzung eine Oberfläche von 80-150 m²/g nach Calcinierung bei 300°C für 5 Stunden aufweist.

6. Verfahren nach einem der vorangehenden Ansprüche, wobei das Atomverhältnis des Metalls der Gruppe IIIB des Periodensystems zu Zink in der Zinkoxidzusammensetzung 1,4 bis 5% ist.

7. Verfahren nach einem der vorangehenden Ansprüche, wobei die Katalysatorzusammensetzung durch die Schritte erhalten wird, umfassend in der Reihenfolge:
(i) Bilden einer Zinkoxidzusammensetzung, die nach Calcinierung bei 300°C für 5 Stunden eine Oberfläche von mindestens 30 m²/g aufweist, wobei die Zinkoxidzusammensetzung im wesentlichen aus Zinkoxid und einem dreiwertigen Metall der Gruppe IIIB des Periodensystems besteht, wobei das Metall in Atomprozent zu dem Zink mit 0,4-15% und in Form von Metall und/oder Metalloxid und/oder einer Verbindung, die thermisch dazu zersetzbar ist und/oder einer Verbindung mit dem Zinkoxid vorliegt, und anschließend
(ii) Dispergieren des Metalls der Gruppe VIII in der Zinkoxidzusammensetzung.

8. Verfahren nach Anspruch 7, wobei das Zinkoxid durch Fällen eines löslichen Zinksalzes aus einer wässerigen Lösung hergestellt wird.

9. Verfahren nach Anspruch 8, wobei das Zinkoxid anschließend calciniert wird.

10. Verfahren nach entweder Anspruch 8 oder Anspruch 9, wobei das Zinkoxid mit dem Metall der Gruppe IIIB imprägniert wird.

11. Verfahren nach Anspruch 8, wobei das Zinkoxid und das Metall der Gruppe IIIB gemeinsam gefällt werden.

12. Verfahren nach einem der Ansprüche 7 bis 11, wobei das Metall der Gruppe VIII durch Imprägnierung auf der Zinkoxidzusammensetzung dispergiert wird.

13. Verfahren nach einem der Ansprüche 7 bis 12, wobei der Katalysator vor der Verwendung in der Fischer-Tropsch-Reaktion unter Bildung von mindestens etwas Metall der Gruppe VIII (oder Metalloxid geringerer Wertigkeit) durch Reduktion mit Wasserstoff oder einem Wasserstoff-reichen Gemisch mit Kohlenmonoxid aktiviert wird.

14. Verfahren nach einem der Ansprüche 7-13, wobei das Metall der Gruppe VIII Cobalt ist.

15. Verfahren nach einem der vorangehenden Ansprüche, wobei Fischer-Tropsch-Bedingungen einen erhöhten Druck im Bereich von 0,1 bis 10 MPa und eine erhöhte Temperatur im Bereich von 180 bis 250°C bedeuten.

## Revendications

1. Procédé pour la conduite d'une réaction de Fischer-Tropsch afin de transformer un mélange de monoxyde de carbone et d'hydrogène en hydrocarbures, qui comprend le passage, dans des conditions de Fischer-Tropsch, dudit mélange sur une composition de catalyseur comprenant un métal du Groupe VIII fixé sur un support consistant en une composition d'oxyde de zinc, composition d'oxyde de zinc qui, après calcination à 300°C pendant 5 heures, a une surface spécifique d'au moins 30 m²/g, ladite composition d'oxyde de zinc consistant essentiellement en oxyde de zinc et un métal trivalent du Groupe IIIB du Tableau Périodique, ledit métal étant présent en un pourcentage atomique par rapport au zinc de 0,4 à 15 % et étant sous forme de métal et/ou d'oxyde métallique et/ou d'un composé apte à la décomposition thermique en oxyde métallique, et/ou d'un composé avec ledit oxyde de zinc.

2. Procédé suivant la revendication 1, dans lequel le métal trivalent est le gallium ou l'aluminium.

3. Procédé suivant la revendication 2, dans lequel le métal est l'aluminium.

4. Procédé suivant la revendication 2, dans lequel l'aluminium ou le gallium est présent au moins partiellement sous forme d'un spinelle avec l'oxyde de zinc.

5. Procédé suivant l'une quelconque des revendications précédentes, dans lequel la composition d'oxyde de zinc a une surface spécifique de 80 à 150 m²/g après calcination à 300°C pendant 5 heures.

6. Procédé suivant l'une quelconque des revendications précédentes, dans lequel la proportion atomique du métal du Groupe IIIB du Tableau Périodique, par rapport au zinc dans la composition d'oxyde de zinc, est égale à une valeur de 1,4 à 5 %.

7. Procédé suivant l'une quelconque des revendications précédentes, dans lequel la composition de catalyseur est obtenue par les étapes consistant successivement :
(i) à former une composition d'oxyde de zinc qui, après calcination à 300°C pendant 5 heures, a une surface spécifique d'au moins 30 m²/g, ladite composition d'oxyde de zinc consistant essentiellement en oxyde de zinc et un métal trivalent du Groupe IIIB du Tableau Périodique, le métal étant présent en un pourcentage atomique par rapport au zinc égal à une valeur de 0,4 à 15 % et étant sous forme de métal et/ou d'un oxyde métallique et/ou d'un composé apte à la décomposition thermique en cet oxyde métallique et/ou d'un composé avec le dioxyde de zinc, puis
(ii) à disperser le métal du Groupe VIII dans la composition d'oxyde de zinc.

8. Procédé suivant la revendication 7, dans lequel l'oxyde de zinc est préparé par précipitation à partir d'une solution aqueuse d'un sel de zinc soluble.

9. Procédé suivant la revendication 8, dans lequel l'oxyde de zinc est ensuite calciné.

10. Procédé suivant la revendication 8 ou la revendication 9, dans lequel l'oxyde de zinc est imprégné avec le métal du groupe IIIB.

11. Procédé suivant la revendication 8, dans lequel l'oxyde de zinc et le métal du Groupe IIIB sont coprécipités.

12. Procédé suivant l'une quelconque des revendications 7 à 11, dans lequel le métal du Groupe VIII est dispersé sur la composition d'oxyde de zinc par imprégnation.

13. Procédé suivant l'une quelconque des revendications 7 à 12, dans lequel le catalyseur, avant utilisation dans la réaction de Fischer-Tropsch, est activé pour former au moins une certaine quantité de métal du Groupe VIII (ou d'oxyde de métal de plus faible valence) par réduction avec l'hydrogène ou un mélange riche en hydrogène, avec le monoxyde de carbone.

14. Procédé suivant l'une quelconque des revendications 7 à 13, dans lequel le métal du Groupe VIII est le cobalt.

15. Procédé suivant l'une quelconque des revendications précédentes, dans lequel les conditions de Fischer-Tropsch consistent en une pression élevée comprise dans l'intervalle de 0,1 à 10 MPa et une température élevée comprise dans l'intervalle de 180 à 250°C.
